# EUROPEAN PATENT APPLICATION

(11) **EP 4 570 909 A1**
(43) Date of publication of application: **18.06.2025**
(21) Application number: 22859540.1
(22) Date of filing: 28.09.2022
(51) Int. Cl.: C12N 15/115, C12Q 1/6848, C12N 9/12, C12Q 1/6806

(54) **PHOTORESPONSIVE NUCLEIC ACID APTAMER, PHOTOACTIVABLE DNA POLYMERASE, AND USE THEREOF**

(30) Priority: 10.08.2022 CN 202210953701
(71) Applicant: Sun Yat-Sen University, Guangzhou, Guangdong 510275 (CN); Shenzhen Qiyu Biotechnology Co., Ltd., Shenzhen, Guangdong, 518000 (CN)
(72) Inventor: ZHENG, Kewei, Guangzhou, Guangdong 510275 (CN); CAI, Tingting, Guangzhou, Guangdong 510275 (CN); WANG, Chenglin, Guangzhou, Guangdong 510275 (CN); CHEN, Yaoqing, Guangzhou, Guangdong 510275 (CN); WANG, Jiaqi, Guangzhou, Guangdong 510275 (CN); ZHONG, Cailing, Guangzhou, Guangdong 510275 (CN)
(74) Representative: Scholl, Matthias
(86) International application number: PCT/CN2022/121959
(87) International publication number: WO 2024/031801

(57) **Abstract**

The disclosure provides a light-responsive aptamer for regulating the activity of a DNA polymerase. The DNA polymerase includes a G-quadruplex-binding peptide and a polymerase sequence; the polymerase sequence includes an active site; and the light-responsive aptamer includes an aptamer sequence and a photoactive linker connected to the aptamer sequence; the aptamer sequence includes a G-quadruplex-forming sequence and a substrate DNA sequence; the G-quadruplex-forming sequence forms a G-quadruplex, and the substrate DNA sequence folds to form a hairpin structure; the G-quadruplex-forming sequence binds to the G-quadruplex-binding peptide, and the substrate DNA binds to the active site of the DNA polymerase; in the presence of ultraviolet light irradiation (UV irradiation), the photoactive linker is broken, and the substrate DNA is detached from the active site of the DNA polymerase.

## Description

The disclosure relates to the field of biotechnology, and more particularly, to a light-responsive aptamer, a light-start DNA polymerase comprising the same, a method for preparing the same and use thereof.

One of the key concerns in nucleic acid detection technology is the formation of the non-specific amplification products caused by primer mismatch and non-template amplification between primers. The non-specific amplification hinders or reduces the amplification of target DNAs, thus decreasing the sensitivity of the nucleic acid detection. Conventionally, there are just a few methods to reduce the non-specific amplification in an isothermal amplification reaction.

Light is a controllable physical signal. Developing a light-responsive aptamer to bind to a G-quadruplex-binding peptide and thus controlling the activity of a light-start DNA polymerase is a strategy to prevent non-specific amplification.

The first objective of the disclosure is to provide a light-responsive aptamer.

The disclosure provides a light-responsive aptamer for regulating an activity of a DNA polymerase; the DNA polymerase comprises a G-quadruplex-binding peptide and a polymerase sequence; the polymerase sequence comprises an active site; and the light-responsive aptamer comprises an aptamer sequence and a photoactive linker connected to the aptamer sequence; the aptamer sequence comprises a G-quadruplex-forming sequence and a substrate DNA sequence; the G-quadruplex-forming sequence forms a G-quadruplex, and the substrate DNA sequence folds to form a hairpin structure; the G-quadruplex-forming sequence binds to the G-quadruplex-binding peptide, and the substrate DNA sequence binds to the active site of the DNA polymerase; in the presence of ultraviolet light irradiation (UV irradiation), the photoactive linker is broken, and the substrate DNA is detached from the active site of the DNA polymerase.

The second objective of the disclosure is to provide a light-start DNA polymerase; the light-start DNA polymerase comprises the DNA polymerase and the light-responsive aptamer;
the DNA polymerase comprises a polymerase sequence and a G-quadruplex-binding peptide;
the light-responsive aptamer comprises an aptamer sequence and a photoactive linker connected to the aptamer sequence; the aptamer sequence comprises a G-quadruplex-forming sequence and a substrate DNA sequence; the G-quadruplex-forming sequence binds to the G-quadruplex-binding peptide, and the substrate DNA sequence binds to the active site of the DNA polymerase to inhibit the activity of the DNA polymerase; in the presence of ultraviolet light irradiation (UV irradiation), the photoactive linker is broken, and the competing substrate DNA is detached from the active site of the DNA polymerase, thereby activating the DNA polymerase.

The third objective of the disclosure is to provide a method for preparing the light-start DNA polymerase, and the method comprises:
dissolving the light-responsive aptamer in a first buffer to a final concentration of 15 - 25 µM thereby obtaining a solution; heating the solution at 92°C - 95°C for 4 min - 6 min; cooling the solution to 23°C - 27°C at a cooling rate of 0.05°C/s - 2°C/s, thereby forming a first mixture;
dissolving the DNA polymerase in a second buffer to a final concentration of 7 - 15 µM, thereby forming a second mixture;
mixing the first mixture with the second mixture in a ratio of 1: 1 to form a third mixture; and resting the third mixture at 4°C for 25 - 35 min, thereby forming a DNA-protein complex; and
mixing equal volumes of glycerol and the DNA-protein complex to form a light-start DNA polymerase.

The fourth objective of the disclosure is to provide a method for activating the light-start DNA polymerase, and the method comprises:
irradiating the light-start DNA polymerase with UV light at a temperature, thereby activating the light-start DNA polymerase; the UV light has a wavelength of 300 nm - 380 nm and irradiates the light-start DNA polymerase for at least 0.5 min.

The fifth objective of the disclosure is to provide a method for synthesizing or detecting a nucleic acid comprising applying the light-start DNA polymerase.

The following advantages are associated with the light-start DNA polymerase and preparation method thereof of the disclosure:

The method provides an efficient and straightforward solution to non-specific amplification and inconsistency of amplification initiation through the activity control of DNA polymerase. For DNA amplifications that reacted above 60°C, such as PCR and LAMP, the hot-start approach is the most popular way to prevent non-specific amplification. The strategies for constructing hot-start DNA polymerases includes blocking with specific antibodies or aptamers, covalent modification, and amino acid mutations. The activity of these hot-start DNA polymerases is inhibited at low temperatures and recovered immediately upon heating to the reaction temperature, thus avoiding non-specific amplification that occurs during the low-temperature stage.
FIG. **1** is a flow chart that illustrates a process for controlling the activity of a DNA polymerase containing a G-quadruplex-binding peptide by a light-responsive aptamer according to one example of the disclosure;
FIG. **2** **is** a map of a recombinant plasmid pCold-I-G4P-Bst according to Example **1** of the disclosure;
FIG. **3A** shows a structure of a G4P-Bst polymerase according to Example **1** of the disclosure; where G4P-Bst represents a large fragment of a Bst polymerase fused at its N-terminus with a G-quadruplex binding protein (G4P);
FIG. **3B** shows a secondary structure of a light-responsive aptamer according to Example **1** of the disclosure;
FIG. **3C** shows the results of primer extension assay in which a light-start G4P-Bst polymerase is irradiated with or without UV light according to Example **2** of the disclosure;
FIG. **3D** is a graph showing the relative activity of the light-start G4P-Bst polymerase in FIG. **3C****;**
FIG. **3E** is shows the cleavage of a light-responsive aptamer in a light-start G4P-Bst polymerase irradiated with UV light for different times according to Example **2** of the disclosure;
FIG. **3F** shows the activity of a light-start G4P-Bst polymerase irradiated with UV light for different times according to Example **2** of the disclosure;
FIG. **4A** shows the position of a PC-linker in a light-response aptamer according to Example **3** of the disclosure;
FIG. **4B** shows the results of a primer extension assay in which a light-start G4P-Bst polymerase is irradiated without UV light (UV-) or with UV light (UV+) for 2 minutes according to Example **3** of the disclosure;
FIG. **4C** shows four light-responsive aptamers each comprising a hairpin structure with a base-paired stem of varying length according to Example 3 of the disclosure;
FIG. **4D** shows the results of a primer extension assay in which a light-start G4P-Bst polymerase is irradiated without UV light (UV-) or with UV light (UV+) for 2 minutes according to Example **3** of the disclosure;
FIG. **5** is a map of a recombinant plasmid pCold-I-G4P-Taq according to Example **4** of the disclosure;
FIG. **6** is a map of a recombinant plasmid pCold-I-G4P-Bsu according to Example **4** of the disclosure;
FIG. **7A** is a flow chart that illustrates a process for controlling the activities of a G4P-Taq polymerase and a G4P-Bsu polymerase by a light-responsive aptamer according to Example **4** of the disclosure;
FIG. **7B** illustrates the results of a primer extension assay for determining the activity of a light-start G4P-Taq polymerase; where CTRL denotes a sample amplified by a G4P-Taq polymerase; UV (-) denotes a sample amplified by a light-start G4P-Taq polymerase without UV irradiation; and UV(+) denotes a sample amplified by a light-start G4P-Taq polymerase irradiated with UV light for 2 minutes;
FIG. **7C** shows the results of a primer extension assay for determining the activity of a light-start G4P-Bsu polymerase; where CTRL denotes a sample amplified by a G4P-Bsu polymerase; UV (-) denotes a sample amplified by a light-start G4P-Bsu polymerase without UV irradiation; and UV(+) denotes a sample amplified by a light-start G4P-Bsu polymerase irradiated with UV light for 2 minutes;
FIG. **8A** is a flow chart that illustrates a process for performing a loop-mediated isothermal amplification (LAMP) reaction by using a heating device according to Example **5** of the disclosure; where the LAMP reaction is carried out on a heating block with a 360-370 nm LED light source at a height of 0.5 cm above a reaction tube cap;
FIG. **8B** shows the results of a colorimetric LAMP assay for detecting E6 and E7 genes of human papillomavirus (HPV) type 45 according to Example **5** of the disclosure;
FIG. **8C** shows the LAMP products of HPV45 DNA in the presence of G4P-Bst (CTRL) or a light-start G4P-Bst polymerase using electrophoresis according to Example **5** of the disclosure.; and
FIGS. **9A-9C** show the results of PCR amplification using a light-start Taq polymerase according to Example **6** of the disclosure; where, in FIG. **9A****,** the samples are amplified by a G4P- Taq polymerase and marked as CTRL; in FIG. **9B****,** the samples are amplified by a light-start G4P-Taq polymerase without UV irradiation and marked as UV (-); and the samples in FIG. **9C** are amplified by a light-start G4P-Taq polymerase irradiated with UV light for 2 minutes and marked as UV(+).

To further illustrate, embodiments detailing a light-responsive aptamer, a light-start DNA polymerase comprising the same, a method for preparing the same and use thereof are described below. It should be noted that the following embodiments are intended to describe and not to limit the disclosure.

The disclosure provides a light-responsive aptamer for regulating the activity of a DNA polymerase; the DNA polymerase comprises a G-quadruplex-binding peptide and a polymerase sequence; the polymerase sequence comprises an active site; and the light-responsive aptamer comprises an aptamer sequence and a photoactive linker connected to the aptamer sequence; the aptamer sequence comprises a G-quadruplex-forming sequence and a substrate DNA sequence; the G-quadruplex-forming sequence forms a G-quadruplex, and the substrate DNA sequence folds to form a hairpin structure; the G-quadruplex-forming sequence binds to the G-quadruplex-binding peptide, and the substrate DNA binds to the active site of the DNA polymerase; in the presence of ultraviolet light irradiation (UV irradiation), the photoactive linker is broken, and the substrate DNA is detached from the active site of the DNA polymerase.

In an alternative preferred embodiment, the G-quadruplex comprises a nucleic acid sequence as shown in **SEQ ID NO:** 1, and/or,
the chemical formula of the photoactive linker is shown as follows:

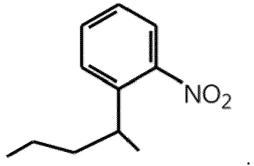

The photoactive linker is attached to its upstream and downstream bases as follows:

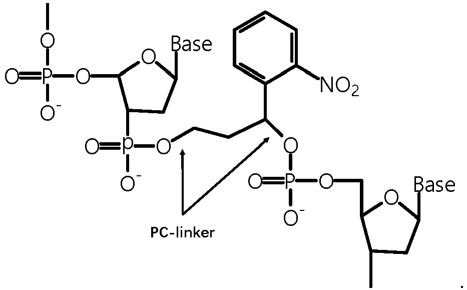

In an alternative preferred embodiment, the substrate DN sequence forms a hairpin structure that comprises a base-paired stem of 5 bp - 20 bp.

In an alternative preferred embodiment, when the base-paired stem has a length of 5 bp - 8 bp, the light-responsive aptamer inhibits the DNA polymerase at 15°C - 40°C.

In an alternative preferred embodiment, when the base-paired stem has a length of 9 bp - 20 bp, the light-responsive aptamer inhibits the DNA polymerase at 15°C - 65°C.

In an alternative preferred embodiment, the G-quadruplex comprises a first loop, a second loop, and a third loop; the hairpin structure comprises a fourth loop;

The photoactive linker is attached to the first loop, the second loop, the third loop, or the fourth loop, or between the hairpin structure and the G-quadruplex.

In an alternative preferred embodiment, when the photoactive linker is disposed between the hairpin structure and the G-quadruplex, under the irradiation of UV light, the DNA polymerase is activated at temperatures of 30°C - 65°C.

In an alternative preferred embodiment, when the photoactive linker is attached to the first loop, the second loop, the third loop, or the fourth loop, under the irradiation of UV light, the DNA polymerase is activated at temperatures of 50°C - 65°C.

The disclosure further provides a light-start DNA polymerase that comprises the DNA polymerase and the light-responsive aptamer;
the DNA polymerase comprises a polymerase sequence and a G-quadruplex-binding peptide; the polymerase sequence comprises an active site and an N-terminus binding to the G-quadruplex-binding peptide;
the light-responsive aptamer comprises an aptamer sequence and a photoactive linker connected to the aptamer sequence; the aptamer sequence comprises a G-quadruplex-forming sequence and a substrate DNA sequence; the G-quadruplex-forming sequence binds to the G-quadruplex-binding peptide, and the substrate DNA sequence binds to the active site of the DNA polymerase to inhibit the activity of the DNA polymerase; in the presence of ultraviolet light irradiation (UV irradiation), the photoactive linker is disconnected from the aptamer sequence, and the substrate DNA sequence is detached from the active site of the DNA polymerase, thereby activating the DNA polymerase.

In an alternative preferred embodiment, the G-quadruplex-binding peptide comprises an amino acid sequence shown in **SEQ ID NO: 2.**

In an alternative preferred embodiment, the polymerase sequence is any one of amino acid sequences of Bst polymerase, Bsu polymerase, and Taq polymerase.

In an alternative preferred embodiment, the polymerase sequence is an amino acid sequence of Bst polymerase as shown in **SEQ ID NO: 3;**
the polymerase sequence is an amino acid sequence of Bsu polymerase as shown in **SEQ ID NO: 4;** or
the polymerase sequence is an amino acid sequence of Taq polymerase as shown in **SEQ ID NO: 5.**

The disclosure further provides a method for preparing the light-start DNA polymerase, and the method comprises:
dissolving the light-responsive aptamer in a first buffer to a final concentration of 15 - 25 uM; heating the solution at 92°C - 95°C for 4 min - 6 min; cooling the solution to 23°C - 27°C at a cooling rate of 0.05°C/s - 2°C/s, thereby forming a first mixture;
dissolving the DNA polymerase in a second buffer to a final concentration of 7 uM - 15 uM, thereby forming a second mixture;
mixing the first mixture with the second mixture in a ratio of 1: 1 to form a third mixture; and resting the third mixture at 4°C for 25 min - 35 min, thereby forming a DNA-protein complex; and
mixing equal volumes of glycerol and the DNA-protein complex to form a light-start DNA polymerase.

In an alternative preferred embodiment, the first buffer comprises 10 mM Tris-HCI (pH 7.4), 75 mM KCI, 0.5 mM EDTA and 0.2 mg/mL bovine serum albumin; and/or,
the second buffer comprises 10 mM Tris-HCI (pH 7.4), 75 mM KCI, 0.5 mM EDTA, 0.2 mg/mL bovine serum albumin, 1 mM dithiothreitol, 1 mM phenylmethylsulfonyl fluoride, and 1% Triton X-100.

The disclosure further provides a method for activating the light-start DNA polymerase, and the method comprises:
irradiating the light-start DNA polymerase with UV light at a temperature, thereby activating the light-start DNA polymerase; and
the UV light has a wavelength of 300 nm - 380 nm and irradiates the light-start DNA polymerase for at least 0.5 min.

In an alternative preferred embodiment, when the photoactive linker is disposed between the hairpin structure and the G-quadruplex, the DNA polymerase is activated at temperatures of 30°C - 65°C; or
the G-quadruplex comprises a first loop, a second loop, and a third loop; the hairpin structure comprises a fourth loop; the photoactive linker is attached to the first loop, the second loop, the third loop, or the fourth loop; and the DNA polymerase is activated at temperatures of 50°C - 65°C.

The disclosure further provides a method for using the light-start DNA polymerase to synthesize or detect nucleic acids.

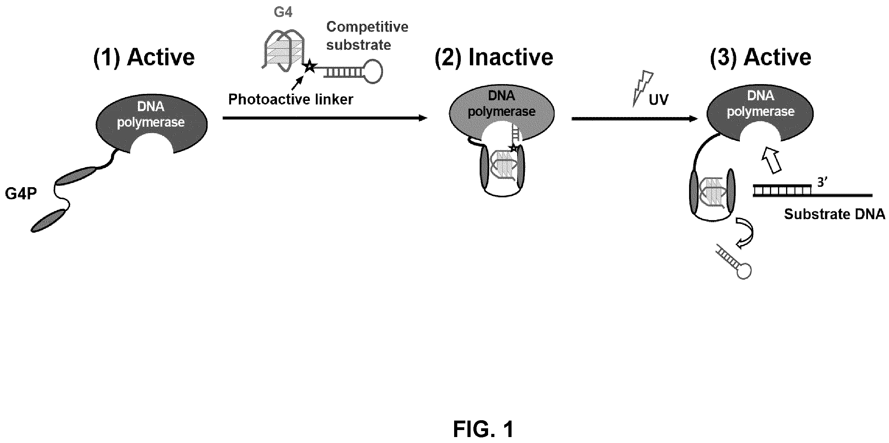

The light-start DNA polymerase comprises the DNA polymerase and the light-responsive aptamer binding to the DNA polymerase. The light-responsive aptamer comprises a G-quadruplex-forming sequence, a substrate DNA sequence, and a photoactive linker (i.e. a PC linker); the G-quadruplex-forming sequence as shown in **SEQ ID NO:** 1 forms a G-quadruplex, and binds to the G-quadruplex-binding peptide; the substrate DNA sequence folds to form a hairpin structure and binds to the active site of the DNA polymerase, so that the light-start DNA polymerase become inactive. When the photoactive linker is cleaved by ultraviolet irradiation, the aptamer sequence is divided into two separate fragments and the substrate DNA sequence (i.e. the hairpin structure) is released from the active site, so that the light-start DNA polymerase become active. FIG. 1 is a flow chart that illustrates a process for controlling the activity of the DNA polymerase by a light-responsive aptamer. The light-start DNA polymerase is activated when the light-responsive aptamer is irradiated with UV light having a specific wavelength. The light-start DNA polymerase is inactive before UV irradiation, so that the non-specific amplification is prevented, increasing the specificity of nucleic acid test. Meanwhile, the light-start G4P-Bst polymerase reduces the non-specific amplification in the LAMP reaction; and the light-start G4P-Taq polymerase improves the success rate of PCR.

### Example 1

Preparation of a G4P-Bst polymerase and a light-start G4P-Bst polymerase.

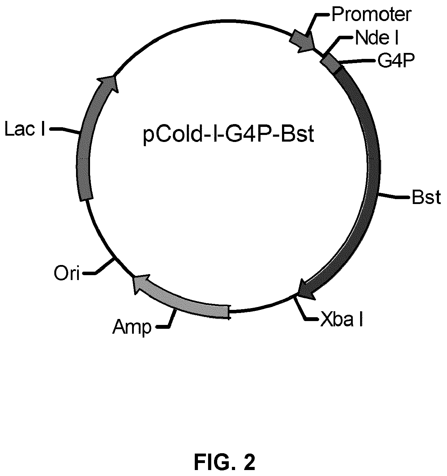

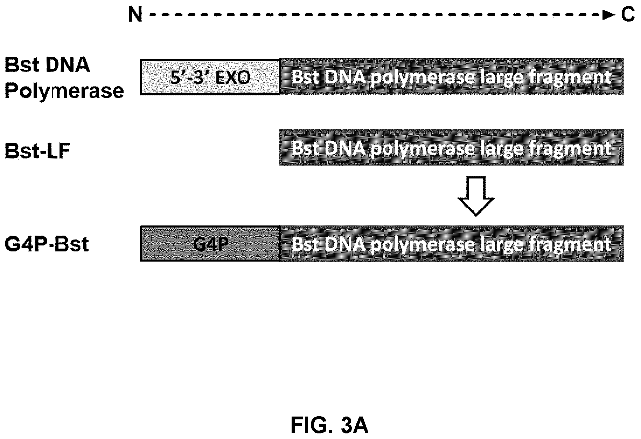

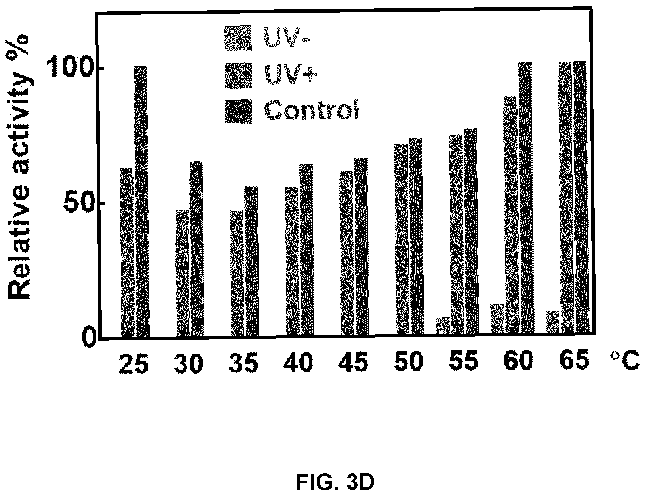

The recombinant plasmid pCold-I-G4P-Bst, as shown in FIG. **2****,** was transformed into E. coli strain BL21 and induced to express a G4P-Bst polymerase; the G4P-Bst polymerase was purified and stored in a buffer solution containing 20 mM Tris-HCI, 150 mM NaCl, 1 mM DTT, 0.5 mM EDTA, and 50 mM % glycerol. FIG. **3A** showed the structures of a Bst polymerase (Bst DNA polymerase), a Bst polymerase lacking the N-terminal 5'-3' exonuclease domain(Bst-LF), and a recombinant Bst polymerase (G4P-Bst).

The Bst-LF was a portion of DNA polymerase | from Bacillus stearothermophilus (ARA98840.1), but lacks a 5'-3' exonuclease domain; the Bst-LF contained 291-878 amino acid residues of the DNA polymerase | as shown in **SEQ ID NO: 3.** The G-quadruplex forming peptide (G4P) was peptide comprising 64 amino acids shown in **SEQ ID NO: 2,** and has a strong binding affinity to G-quadruplex.

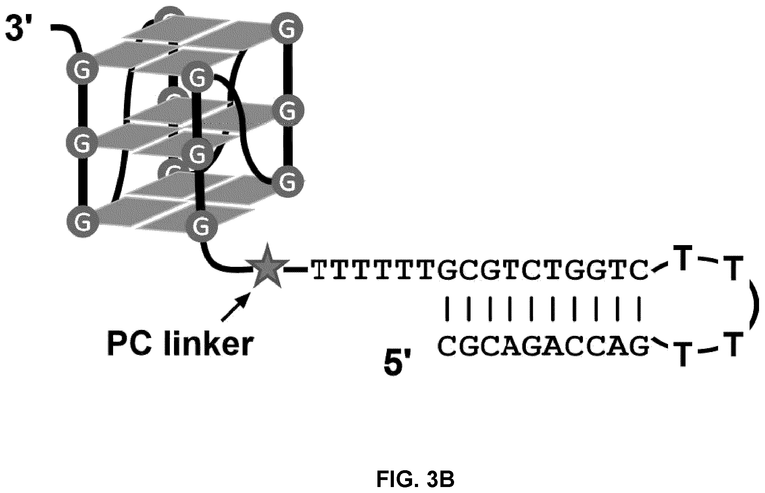

FIG. **3B** showed a secondary structure of a light-responsive aptamer. The light-responsive aptamer (PC-1) shown in Table **1** was dissolved in a first buffer to a final concentration of 20 µM, heated at 95°C for 5 min, and cooled to 25°C at a cool rate of 0.1 °C/s; the first buffer contained 10 mM Tris-HCI (pH 7.4), 75 mM KCI, 0.5 mM EDTA, and 0.2 mg/mL bovine serum albumin. The G4P-Bst polymerase was dissolved in a second buffer to a final concentration of 10 µM; and the buffer solution contained 10 mM Tris-HCI (pH 7.4), 75 mM KCI, 0.5 mM EDTA, 0.2 mg/mL bovine serum albumin, 1 mM dithiothreitol, 1 mM phenylmethylsulfonyl fluorine, and 1% Triton X-100. The light-responsive aptamer and the G4P-Bst polymerase were mixed in a ratio of 1: 1, and allowed to stand at 4°C for 30 min to form a DNA-protein complex. Equal volumes of glycerol and the DNA-protein complex were mixed to form a light-start DNA polymerase containing G4P-Bst. The light-start DNA polymerase was stored at - 20°C - -30°C.

**Table 1**

| Name | Sequence |
|---|---|
| PC-1 | 5'-CGCAGACCAGTTTTCTGGTCTGCGTTTTTT**(SEQ ID NO: 6)**/PC-linker/TGGGTGGGTGGGTGGGT-3' **(SEQ ID NO: 7)** |

### Example 2

Use of primer extension assay to determine the ability of the light-responsive aptamer to control the light-start DNA polymerase.

The primer extension assay was performed in a 25 µL reaction containing 20 mM Tris-HCI (pH 8.8), 10 mM (NH₄)₂SO4, 50 mM KCI, 8 mM MgSO₄, 0.1% Tween-20, 2.5 mM dNTPs, 100 nM CSTB-A primers, 100 nM template DNA, and 100 nM DNA polymerase. Table **2** listed the sequences of the primers and the substrate DNA. The reaction was performed at 15°C-65°C for 5 min and terminated with four volumes of a stop solution (containing 99% formamide, 0.1% SDS, and 20 mM EDTA). Products were denatured at 95°C for 5 min and electrophoresed on a 12% denaturing polyacrylamide-urea gel in 1×TBE buffer. The primers and full-length products were photographed on a ChemiDoc MP imaging system (Bio-Rad) and quantified using Image Quant 5.2 software.

**Table 2**

| Name | Sequence (5'-3') |
|---|---|
| CSTB-A | 5'-FAM-CCAGCCTGCGGCGAGTG-3' **(SEQ ID NO: 8)** |
| Templat e-GM | 5'- TCTATTACATTCTAAGAGTTAGAGTTAGGGTCTACTCTTCTTCTCTT CTTCACTCGCCGCAGGCTGG-3' (SEQ ID NO: 9) |

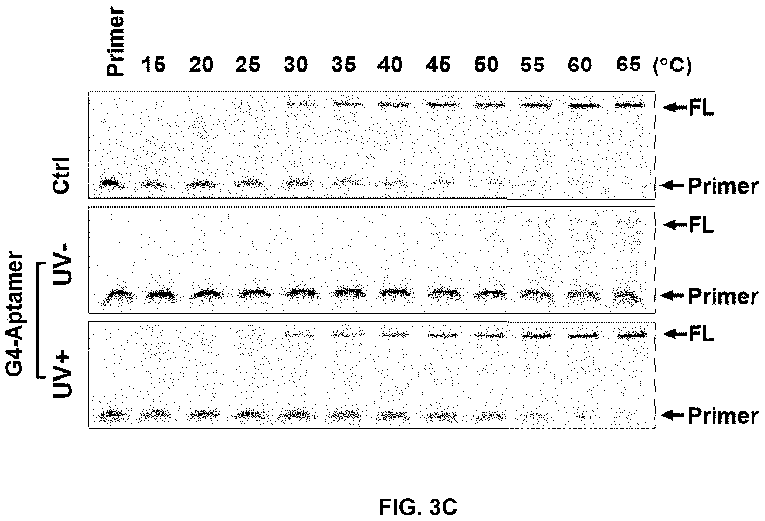

FIGS. **3C-3D** showed the results of primer extension assay for determining the relative activity of the G4P-Bst polymerase. The G4P-Bst polymerase was active at 15°C - 65°C. The light-responsive aptamer (PC-1) bound to the G4P-Bst polymerase to form a light-start G4P-Bst polymerase that was inactive or exhibited a low level of activity at a temperature of greater than 55°C. The light-start G4P-Bst polymerase was fully activated when UV light irradiated the light-responsive aptamer (PC-1).

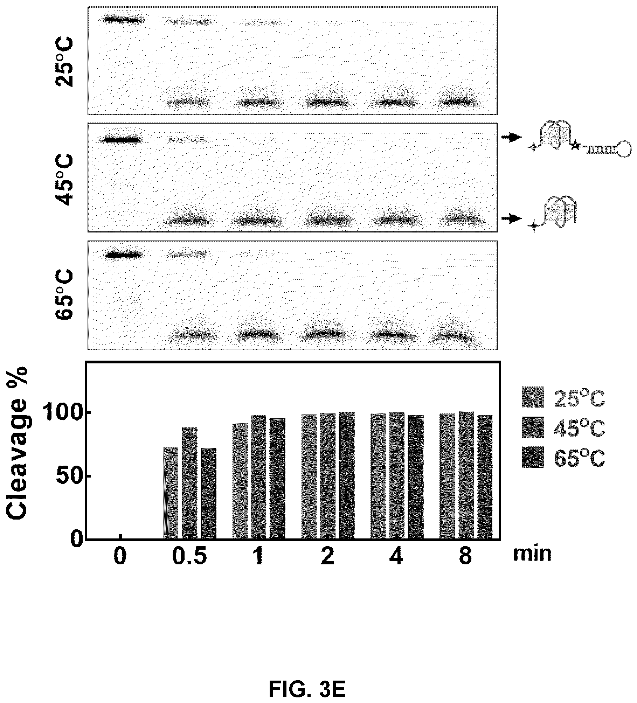

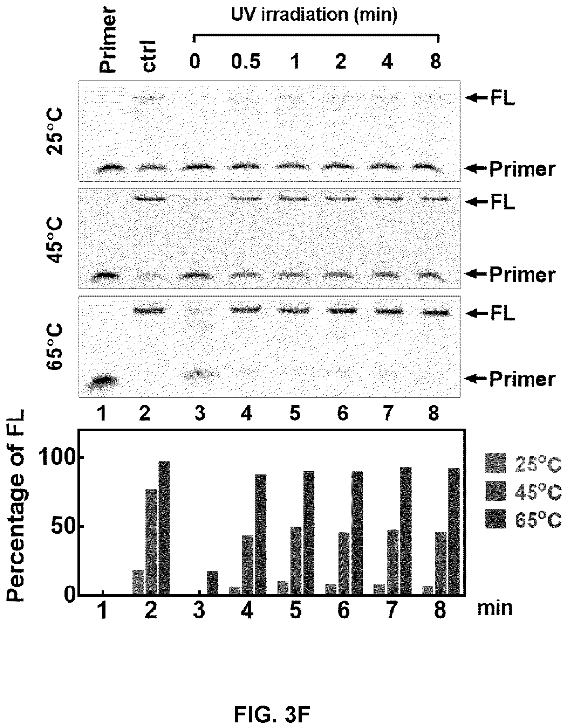

Upon UV irradiation, the polymerase activity was determined by the fragmentation efficiency of the light-responsive aptamer (PC-1). As shown in FIG. 3E, the light-responsive aptamer (PC-1) was mostly fragmented under UV irradiation for 0.5 min, and achieved 100% fragmentation efficiency after 1 minute of UV irradiation. The fragmentation efficiency remained constant at different temperatures. The light-start G4P-Bst polymerase was irradiated with UV light and employed in the primer extension assay; as shown in FIG. **3F****,** the light-start G4P-Bst polymerase become active after 0.5 minutes of UV irradiation and was fully activated after 1 minute of UV irradiation, which was consistent with that in FIG. **3E****.**

The results demonstrated that the light-responsive aptamer (PC-1) bound to the G4P-Bst polymerase to form the light-start G4P-Bst polymerase that was inactive at 15°C - 65°C; and the light-start G4P-Bst polymerase was activated when the UV light irradiated the light-responsive aptamer.

### Example 3

Effect of photo-cleavage site and hairpin stem length on the activity of the DNA polymerase.

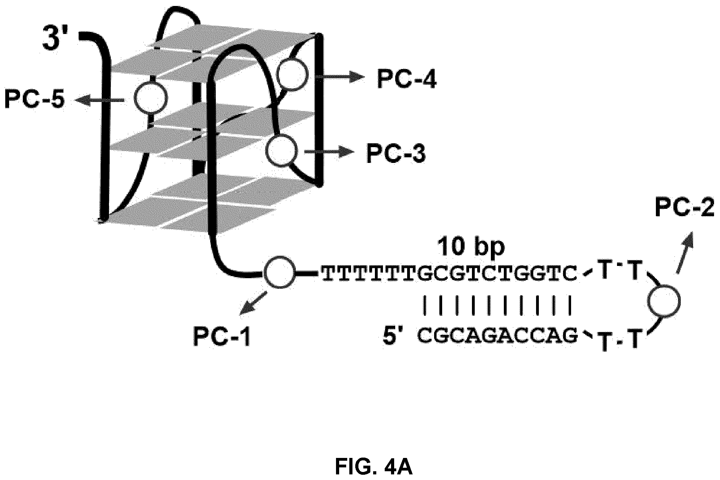

The light-responsive aptamer was modified to have different photo-cleavage sites (FIGS. **4A****),** used to prepare the light-start G4P-Bst polymerase according to the method of Example **2,** and employed in primer extension assay. Table **3** listed the sequences of five light-responsive aptamers comprising different PC-linkers.

**Table 3**

| Nam e | Sequence |
|---|---|
| PC-1 | 5'-CGCAGACCAGTTTTCTGGTCTGCGTTTTTT**(SEQ ID NO: 6)**/PC-linker/TGGGTGGGTGGGTGGGT-3' **(SEQ ID NO: 7)** |
| PC-2 | 5'-CGCAGACCAGTT**(SEQ ID NO: 10)**/ PC-linker /TTCTGGTCTGCG TTTTTTTGGGTGGGTGGGTGGGT-3' **(SEQ ID NO: 11)** |
| PC-3 | 5'-CGCAGACCAGTTTTCTGGTCTGCGTTTTTTTGGG**(SEQ ID NO: 12)**/PC-linker /TGGGTGGGTGGGT-3' **(SEQ ID NO: 13)** |
| PC-4 | 5'-CGCAGACCAGTTTTCTGGTCTGCGTTTTTTTGGGTGGG**(SEQ ID NO: 14)**/PC-linker /TGGGTGGGT-3' |
| PC-5 | 5'-CGCAGACCAGTTTTCTGGTCTGCGTTTTTTTGGGTGGGTGGG**(SE Q ID NO: 15)**/PC-linker / TGGGT-3' |

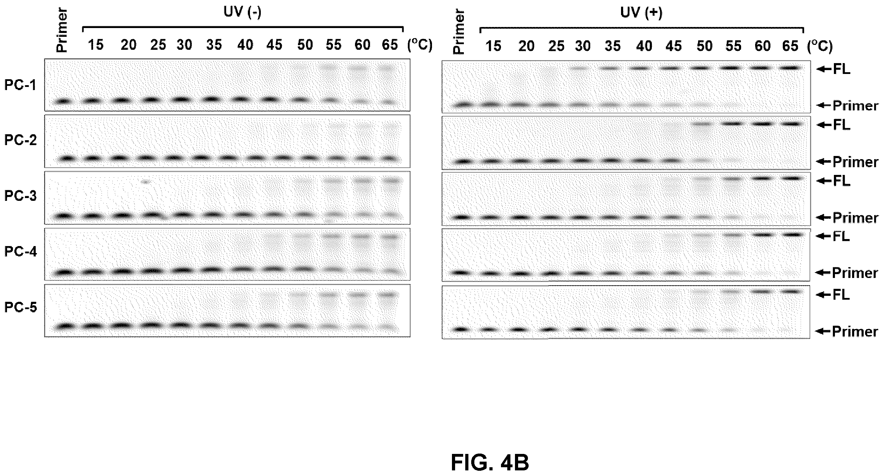

As shown in FIG. **4B****,** the G4P-Bst polymerase was inhibited by any one of the five light-responsive aptamers; Upon UV irradiation, the G4P-Bst polymerase was fully activated at 30°C - 65°C when the light-responsive aptamer (PC-1) contained a PC-linker inserted between the G-quadruplex and the hairpin structure. Under UV irradiation, the G4P-Bst polymerase was activated at a temperature of at least 50°C when the light-responsive aptamer (PC-2, PC-3, PC-4, or PC-5) contained a PC linker binding at the other photo-cleavage sites. The G-quadruplex comprises a first loop, a second loop, and a third loop; the hairpin structure comprises a fourth loop. In the example, the term "other photo-cleavage sites" as used herein refers to the first loop, the second loop, the third loop, and the fourth loop.

The results demonstrated that when the PC linker was inserted between the G-quadruplex and the hairpin structure, the G4P-Bst polymerase was activated by only UV irradiation; when the PC linker was attached to the first loop, the second loop, the third loop, and the fourth loop, the G4P-Bst polymerase was activated by both UV light and temperatures.

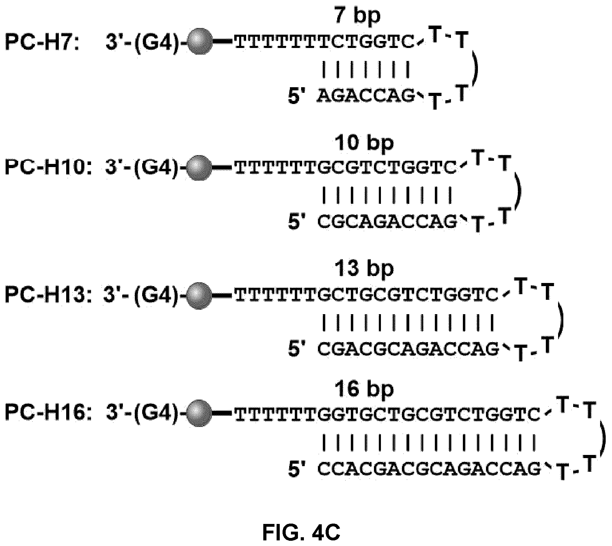

The hairpin structure was modified to have a base-paired stem of varying length (FIG. **4C****),** employed to prepare the light-start G4P-Bst polymerase according to the method of Example **2,** and used in the primer extension assay. Table **4** listed the different sequences of the base-paired stem.

**Table 4**

| Name | Sequence |
|---|---|
| PC-H7 | 5'-AGACCAGTTTTCTGGTCTTTTTTT**(SEQ ID NO: 16)**/ PC-linker /TGGGTGGGTGGGTGGGT-3' **(SEQ ID NO: 17)** |
| PC-H10 | 5'-CGCAGACCAGTTTTCTGGTCTGCGTTTTTT**(SEQ ID NO: 18)**/ PC-linker /TGGGTGGGTGGGTGGGT-3' **(SEQ ID NO: 19)** |
| PC-H13 | 5'-CGACGCAGACCAGTTTTCTGGTCTGCGTCGTTTTTT**(SEQ ID NO: 20)**/PC-linker /TGGGTGGGTGGGTGGGT-3' **(SEQ ID NO: 21)** |
| PC-H16 | 5'-CCACGACGCAGACCAGTTTTCTGGTCTGCGTCGTGGTTTTTT**(SEQ ID NO: 22)**/PC-linker /TGGGTGGGTGGGTGGGT-3' **(SEQ ID NO: 23)** |

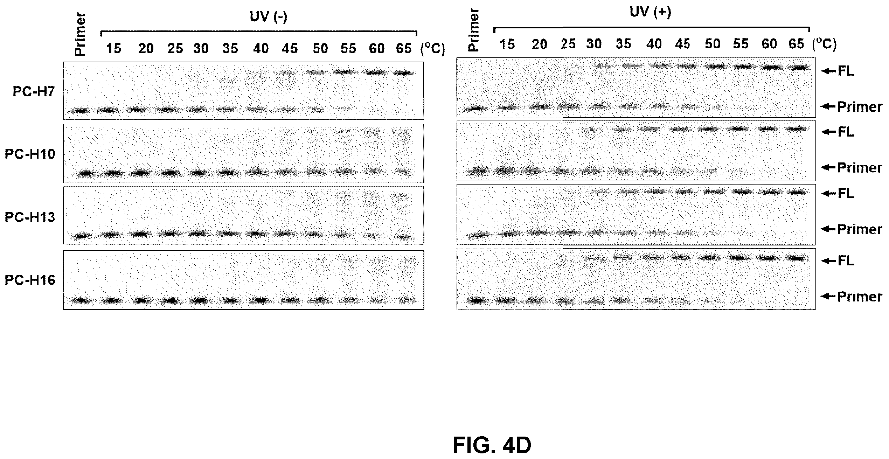

As shown in FIGS. **4D****,** when the base-paired stem was 7bp in length, the light-responsive aptamer inhibited the G4P-Bst polymerase at 15°C - 40°C. When the base-paired stem was 10-16 bp in length, the light-responsive aptamer inhibited the G4P-Bst polymerase at 15°C - 65°C. Upon UV irradiation, each of the five light-responsive aptamers activated the G4P-Bst polymerase at 30°C - 65°C. The results demonstrated that when the base-paired stem was 10-16 bp in length, the G4P-Bst was activated under UV irradiation over a wide range of temperatures.

### Example 4

The combination of G-quadruplex binding peptide and light-responsive G4-aptamer is a versatile strategy for the construction of another light-controlled DNA polymerase.

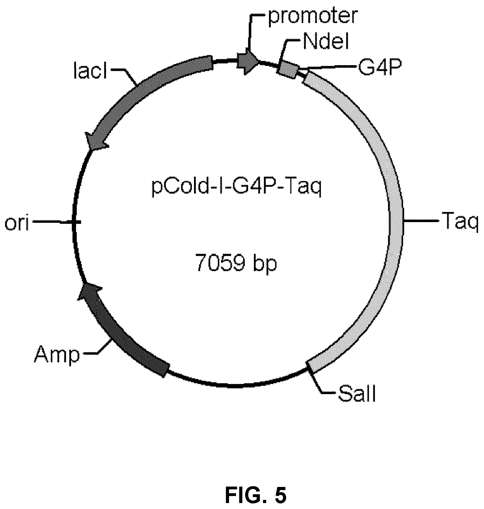

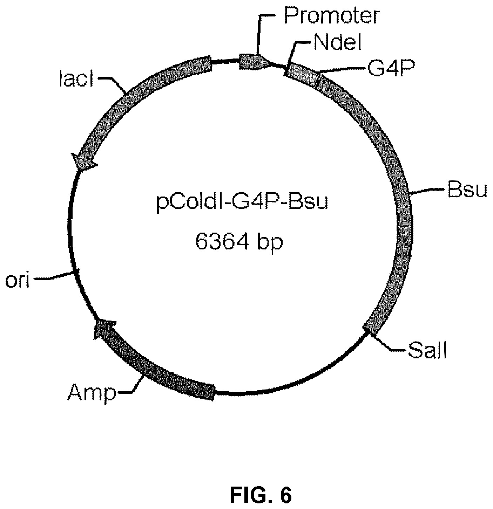

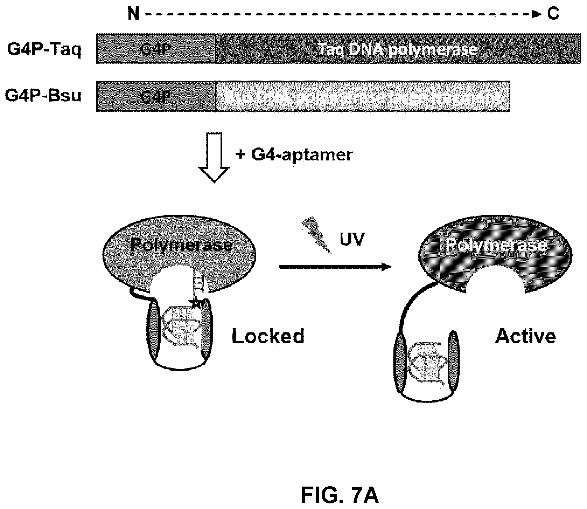

G4P-Taq polymerase and G4P-Bsu polymerase were prepared according to the method of Example **1.** In the recombinant plasmid pCold-I-G4P-Bst, the Bst sequence was replaced by a Taq sequence or Bsu sequence to form two recombinant plasmids pCold-I-G4P-Taq and pCold-I-G4P-Bsu, as shown in FIGS. **5-6****.** FIG. **7A** illustrated a process for controlling the activities of the G4P-Taq polymerase and the G4P-Bsu polymerase by the light-responsive aptamer.

The activity of G4P-Taq polymerase was measured according to the method of Example 2; and G4P-Taq polymerase without the light-responsive aptamer were used as controls; and the primer extension assay was carried out at different temperatures for 15 min. A reaction buffer contained 10 mM Tris-HCI (pH 8.8), 50 mM KCI, 1.5 mM MgCl2, and 0.08% Nonidet P40. A 10 µL reaction contained 2.5 mM dNTP, 100 nM CSTB-A primer, 100 nM template DNA, and 100 nM the G4P-Taq polymerase.

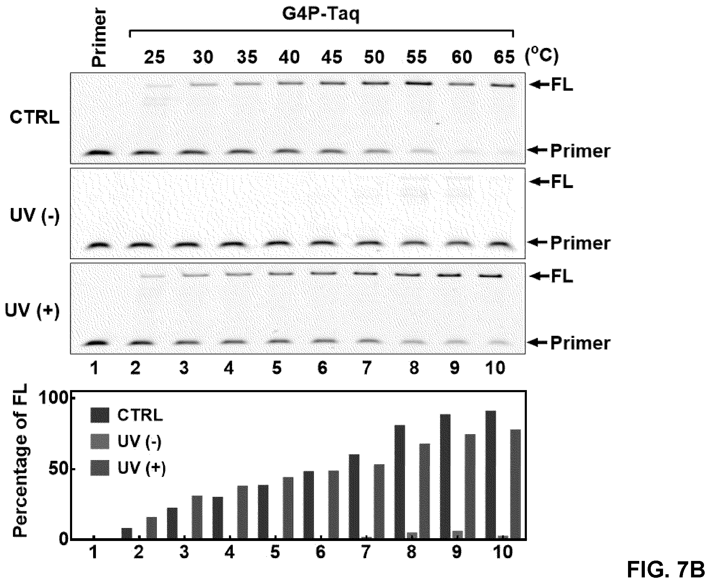

As shown in FIG. **7B****,** the G4P-Taq polymerase was active at 25°C - 65°C; the light-responsive aptamer bound to the G4P-Taq polymerase to form a light-start G4P-Taq polymerase that was inactive at temperatures of lower than 55°C and active at temperature of higher than 55°C; in the primer extension assay, both the light-start G4P-Taq polymerase and the G4P-Taq polymerase were fully activated at 25°C - 65°C under UV irradiation.

The activity of the G4P-Bsu polymerase was measured according to the method of Example **2;** and the samples mixed with the light-responsive aptamer were used as controls; the primer extension assay was carried out at different temperatures for 15 min. A reaction buffer contained 10 mM Tris-HCI (pH 8.8), 50 mM NaCl, 10 mM MgCl₂, and 1 mM DTT. A 10 µL reaction contained 2.5 mM dNTPs, 100 nM CSTB-A primers, 100 nM template DNA, and 100 nM the G4P-Bsu polymerase.

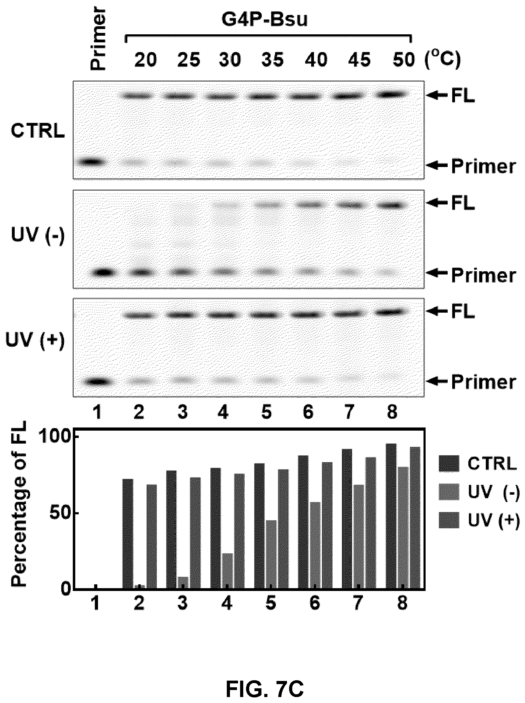

As shown in FIG. **7C****,** the G4P-Bsu polymerase was active at 20°C - 50°C; the light-responsive aptamer bound to the G4P-Bsu polymerase to form a light-start G4P- Bsu polymerase that was inactive in the absence of UV light and at temperatures of lower than 40°C; in the primer extension assay, both the G4P-Bsu polymerase and light-start G4P-Bsu polymerase were fully activated at 20°C - 50°C under UV irradiation.

The results demonstrated that the light-responsive aptamer bound to the G4P-Taq polymerase or G4P-Bsu polymerase to form the light-start DNA polymerase that was activated under UV irradiation.

### Example 5

Detection of E6 and E7 genes of human papillomavirus (HPV) type 45 using the light-start G4P-Bst polymerase.

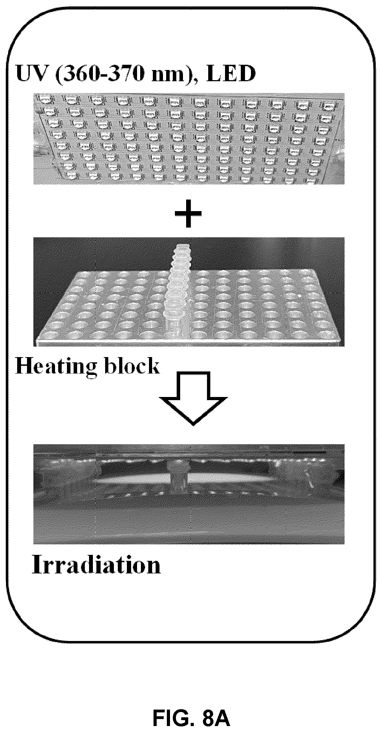

A colorimetric LAMP reaction contained 10 mM (NH₄)₂SO4, 50 mM KCI, 8 mM MgSO₄, 0.1 % Tween-20, 1.6 mM dNTP, 100 nM the G4P-Bst polymerase or the light-start G4P-Bst polymerase, 0.013% (w/V) cresol red, 1.6 µM FIP/BIP primers, 0.4 µM FLP/BLP primers, 0.2 µM F3/B3 primers, and different copies of a recombinant plasmid containing E6 and E7 genes of HPV45. As shown in FIG. **8A****,** the LAMP reaction was carried out in a 65°C dry bath with a 360-370 nm LED light source at a height of 0.5 cm above the reaction tube cap for 0.5-8 min.

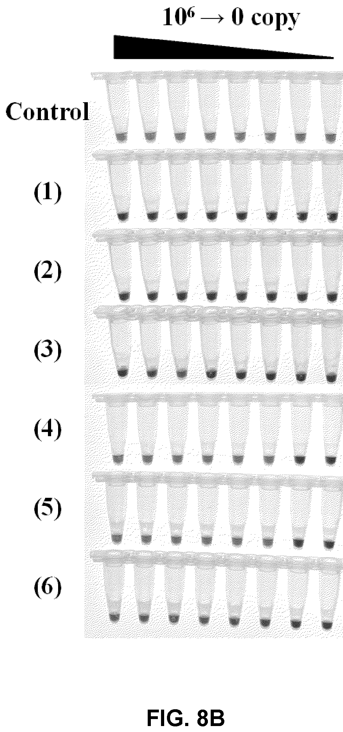

A recombinant plasmid containing the E6 and E7 genes of HPV45 was serially diluted 10-fold, and added to the samples (as shown in FIGS. **8B- 8C****,** the copy numbers of the E6 and E7 genes in the samples from left to right were 1,000,000, 100,000, 10,000, 1,000, 100, 10, 1, and 0); the G4P-Bst polymerase caused the samples to turn from red to yellow (Control group in FIG. **8B****),** which indicated the occurrence of DNA amplification regardless of whether the HPV45 template DNA was present in the LAMP reaction or not; in the absence of UV irradiation, the light-start G4P-Bst polymerase maintained the color of the samples, indicating that the DNA amplification did not occur in the LAMP reaction (Group 1 in FIG. **8B****);** when the UV light irradiated the light-start G4P-Bst polymerase (Groups 2-6 in FIG. **8B****)** for 0.5, 1, 2, 4, and 8 min at an initial stage of the LAMP reaction, significant color changes occurred in the samples that contained more than ten copies of the recombinant plasmid, especially at 2-4 min (Groups 4-5 in FIG. **8B****).**

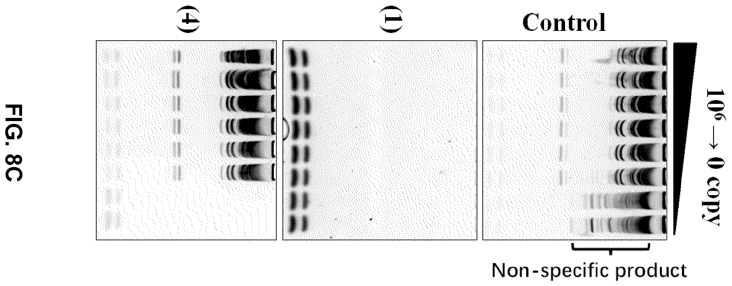

Further, an electrophoretic analysis was performed on the amplified products in the control group, as well as the groups 1 and 4. As shown in FIG. **8C****,** in the control group, specific bands appeared in the absence of the template DNA, and non-specific bands appeared in the lanes loaded with the amplified products containing high concentrations of the template DNA; no DNA bands were observed in the group 1, indicating that the light-start G4P-Bst polymerase was unable to amplify the DNA template in the absence of UV irradiation; in the group 4, similar bands appeared in the lanes that were loaded with the products amplified by the light-start G4P-Bst polymerase under UV irradiation for two minutes. The results showed that the light-start G4P-Bst polymerase was inhibited before UV irradiation and failed to elongate a mismatched primer-template, which is consistent with the results of the colorimetric LAMP assay.

The result demonstrated that the light-start G4P-Bst polymerase prevented the formation of non-specific amplification products, thereby increasing the specificity of the LAMP amplification.

### Example 6

Light-start PCR was performed using the light-start Taq DNA polymerase.

Genomic DNA of Hela cell was used as a template for PCR amplification by which eight specific sites within NPM1 gene were detected. PCR products were analyzed by agarose electrophoresis. Table **5** showed the sequences of primers that bind to the eight specific sites within the NPM1 gene.

**Table 5**

| Primer name | Sequence (5'-3') |
|---|---|
| 259-F | GCTGCTGATGAAGATGATGACG **(SEQ ID NO: 24)** |
| 259-R | TGTAAAGGCTCACAGACCCAATA **(SEQ ID NO: 25)** |
| 371-F | GCCCTTTCCTATGCTTAGATGC **(SEQ ID NO: 26)** |
| 371-R | TTACTTTAATTGGACTGCCTTCGT **(SEQ ID NO: 27)** |
| 462-F | GCCATTTGGGAATTTAGGTAGG **(SEQ ID NO: 28)** |
| 462-R | CCGTTCTTAAAGATAACTGGTGC **(SEQ ID NO: 29)** |
| 562-F | TGTGCCAGTTCTTGGGTTTGTT **(SEQ ID NO: 30)** |
| 562-R | CAGGACCTTGAAGTGATTATTTGTG **(SEQ ID NO: 31)** |
| 648-F | TGTCCCTTTGTTCTGAAGATTTAGTG **(SEQ ID NO: 32)** |
| 648-R | GGCTGGGTGTTAGGATTACGG **(SEQ ID NO: 33)** |
| 771-F | CATGAAGTTGCTGTATTGGGATTT **(SEQ ID NO: 34)** |
| 771 -R | AGCGTTCATAGCAGTCAACAAGTAG **(SEQ ID NO: 35)** |
| 906-F | TGGAGTAGGACCTGATTGTAGTATTG **(SEQ ID NO: 36)** |
| 906-R | CGAACCTTCATGCACCTCTTTG **(SEQ ID NO: 37)** |
| 1350-F | TTTTCGTGGCTTCAGTAGATTCC **(SEQ ID NO: 38)** |
| 1350-R | TGGGCAGCATGGCTAGTATCT **(SEQ ID NO: 39)** |

The PCR reaction contained 10 mM Tris-HCI (pH 8.8), 50 mM KCI, 1.5 mM MgCl2, 250 µM dNTP, 4% DMSO, 40 ng of genomic DNA, 0.8 µM upstream primer, 0.8 µM downstream primer, and 5 nM G4P-Taq polymerase. The PCR temperature cycling conditions were as follows: incubation at 65°C for 3 min, denaturation at 95°C for 5 min; 30 cycles of denaturation at 95°C for 30 s, annealing at 60°C for 30 s, and elongation at 72°C for 1 min; and the final cycle was followed by extension at 72°C for 5 min. During the incubation, the PCR reaction was exposed to UV light.

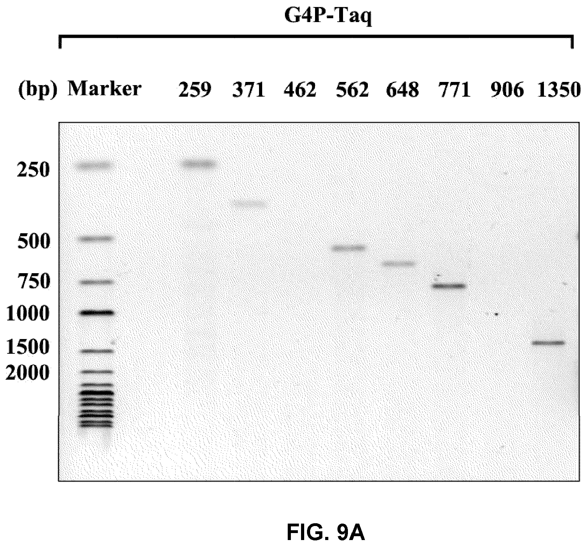

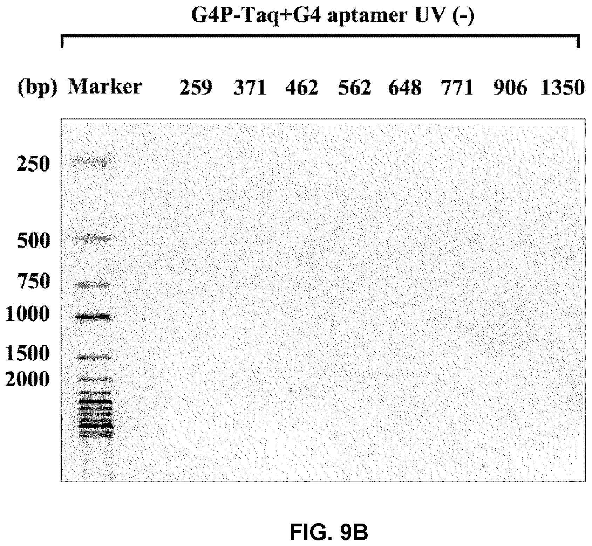

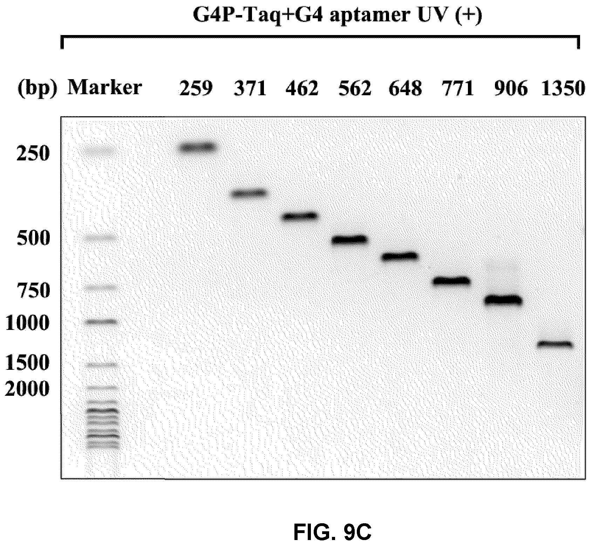

As shown in FIG. **9A****,** the G4P-Taq polymerase caused six bands each representing a DNA fragment at a specific site within the NPM1 gene; no bands appeared (as shown in FIG. **9B****)** after PCR amplification with the light-start G4P-Taq polymerase without UV irradiation; and eight target bands appeared (as shown in FIG. **9C****)** after PCR amplification with the light-start G4P-Taq polymerase under UV irradiation.

The results demonstrated that the light-responsive aptamer bound to the G4P-Taq polymerase so as to block the polymerase activity, thus inhibiting PCR amplification; the competitive substrates were released from the active site when the photoactive linker was cleaved by UV irradiation, so that the G4P-Taq polymerase was fully activated. The light-start G4P-Bst polymerase was inhibited prior to UV irradiation and hence failed to elongate a mismatched primer-template.

The results demonstrated that the light-responsive aptamer bound to the DNA polymerase comprising the G-quadruplex-binding peptide to form a light-start DNA polymerase that was activated under UV light.

It will be obvious to those skilled in the art that changes and modifications may be made, and therefore, the aim in the appended claims is to cover all such changes and modifications.

## Claims

1. A light-responsive aptamer for regulating an activity of a DNA polymerase; the DNA polymerase comprising a G-quadruplex-binding peptide and a polymerase sequence; the polymerase sequence comprising an active site; and the light-responsive aptamer comprising an aptamer sequence and a photoactive linker connected to the aptamer sequence; wherein the aptamer sequence comprises a G-quadruplex-forming sequence and a substrate DNA sequence; the G-quadruplex-forming sequence forms a G-quadruplex, and the substrate DNA sequence folds to form a hairpin structure; the G-quadruplex-forming sequence binds to the G-quadruplex-binding peptide, and the substrate DNA binds to the active site of the DNA polymerase; in the presence of ultraviolet light irradiation (UV irradiation), the photoactive linker is broken, and the substrate DNA is detached from the active site of the DNA polymerase.

2. The light-responsive aptamer of claim 1, wherein the G-quadruplex comprises a nucleic acid sequence as shown in **SEQ ID NO: 1,** and/or, a chemical formula of the photoactive linker is shown as follows:

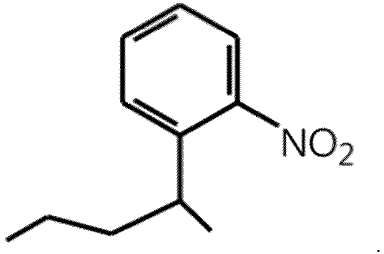

3. The light-responsive aptamer of claim 1, wherein the substrate DNA sequence forms a hairpin structure that comprises a base-paired stem having a length of 5 bp - 20 bp.

4. The light-responsive aptamer of claim 3, wherein when the base-paired stem has a length of 5 bp - 8 bp, the light-responsive aptamer inhibits the DNA polymerase at 15°C - 40°C.

5. The light-responsive aptamer of claim 3, wherein when the base-paired stem has a length of 9 bp - 20 bp, the light-responsive aptamer inhibits the DNA polymerase at 15°C - 65°C.

6. The light-responsive aptamer of claim 1, wherein the G-quadruplex comprises a first loop, a second loop, and a third loop; the hairpin structure comprises a fourth loop; and the photoactive linker is attached to the first loop, the second loop, the third loop, or the fourth loop, or between the hairpin structure and the G-quadruplex.

7. The light-responsive aptamer of claim 6, wherein when the photoactive linker is disposed between the hairpin structure and the G-quadruplex, under the irradiation of UV light, the DNA polymerase is activated at temperatures of 30°C - 65°C.

8. The light-responsive aptamer of claim 6, wherein when the photoactive linker is attached to the first loop, the second loop, the third loop, or the fourth loop, under the irradiation of UV light, the DNA polymerase is activated at temperatures of 50°C - 65°C.

9. A light-start DNA polymerase, comprising a DNA polymerase and the light-responsive aptamer of claim 1; wherein the DNA polymerase comprises a polymerase sequence and a G-quadruplex-binding peptide; the polymerase sequence comprises an active site and an N-terminus binding to the G-quadruplex-binding peptide;
the light-responsive aptamer comprises an aptamer sequence and a photoactive linker connected to the aptamer sequence; the aptamer sequence comprises a G-quadruplex-forming sequence and a substrate DNA sequence; the G-quadruplex-forming sequence binds to the G-quadruplex-binding peptide, and the substrate DNA binds to the active site of the DNA polymerase to inhibit the activity of the DNA polymerase; in the presence of ultraviolet light irradiation (UV irradiation), the photoactive linker is broken, and the substrate DNA is detached from the active site of the DNA polymerase, thereby activating the DNA polymerase.

10. The light-start DNA polymerase of claim 9, wherein the G-quadruplex-binding peptide comprises an amino acid sequence shown in **SEQ ID NO: 2.**

11. The light-start DNA polymerase of claim 9, wherein the polymerase sequence is any one of amino acid sequences of Bst polymerase, Bsu polymerase, and Taq polymerase.

12. The light-start DNA polymerase of claim 11, wherein:
the polymerase sequence is an amino acid sequence of Bst polymerase as shown in **SEQ ID NO: 3;**
the polymerase sequence is an amino acid sequence of Bsu polymerase as shown in **SEQ ID NO: 4;** or
the polymerase sequence is an amino acid sequence of Taq polymerase as shown in **SEQ ID NO: 5.**

13. A method for preparing the light-start DNA polymerase of claim 9, the method comprising:
dissolving the light-responsive aptamer in a first buffer to a final concentration of 15 - 25 µM thereby obtaining a solution; heating the solution at 92°C - 95°C for 4 min - 6 min; cooling the solution to 23°C - 27°C at a cooling rate of 0.05°C/s - 2°C/s, thereby forming a first mixture;
dissolving the DNA polymerase in a second buffer to a final concentration of 7 - 15 µM, thereby forming a second mixture;
mixing the first mixture with the second mixture in a ratio of 1: 1 to form a third mixture; and resting the third mixture at 4°C for 25 min - 35 min, thereby forming a DNA-protein complex; and
mixing equal volumes of glycerol and the DNA-protein complex to form a light-start DNA polymerase.

14. The method of claim 13, wherein
the first buffer comprises 10 mM Tris-HCI (pH 7.4), 75 mM KCI, 0.5 mM EDTA and 0.2 mg/mL bovine serum albumin; and/or,
the second buffer comprises 10 mM Tris-HCI (pH 7.4), 75 mM KCI, 0.5 mM EDTA, 0.2 mg/mL bovine serum albumin, 1 mM dithiothreitol, 1 mM phenylmethylsulfonyl fluoride, and 1% Triton X-100.

15. A method for activating the light-start DNA polymerase of claim 9, and the method comprising: irradiating the light-start DNA polymerase with UV light at a temperature, thereby activating the light-start DNA polymerase; wherein
the UV light has a wavelength of 300 nm - 380 nm and irradiates the light-start DNA polymerase for at least 0.5 min.

16. The method of claim 15, wherein:
when the photoactive linker is disposed between the hairpin structure and the G-quadruplex, the DNA polymerase is activated at temperatures of 30°C - 65°C; or
the G-quadruplex comprises a first loop, a second loop, and a third loop; the hairpin structure comprises a fourth loop; the photoactive linker is attached to the first loop, the second loop, the third loop, or the fourth loop; and the DNA polymerase is activated at temperatures of 50°C - 65°C.

17. A method for synthesizing or detecting a nucleic acid comprising applying the light-start DNA polymerase of claim 9.
